(19) Europäisches Patentamt
European Patent Office
Office européen des brevets

(11) Publication number: **0 168 908**
**B1**

# (12) EUROPEAN PATENT SPECIFICATION

(45) Date of publication of patent specification: **07.12.88**

(51) Int. Cl.[4]: **C 07 C 103/38,** C 07 C 131/00, C 07 C 45/46

(21) Application number: **85302452.9**

(22) Date of filing: **04.04.85**

## (54) Process for producing N-acyl-acyloxy aromatic amines.

(30) Priority: **04.06.84 US 616989**
**08.06.84 US 618659**
**03.07.84 US 627381**
**03.07.84 US 627382**
**21.08.84 US 642981**

(43) Date of publication of application:
**22.01.86 Bulletin 86/04**

(45) Publication of the grant of the patent:
**07.12.88 Bulletin 88/49**

(84) Designated Contracting States:
**AT BE CH DE FR GB IT LI LU NL SE**

(56) References cited:
**CHEMICAL ABSTRACTS, vol. 99, no. 1, 4th July 1983, page 482, no. 5285a, Columbus, Ohio, US; R. BUTLER et al.: "Direct detection of intermediates and synthetic applications of the reaction of thionyl chloride with oximes of substituted acetophenones and benzaldehydes: Beckmann rearrangements"**

**JOURNAL OF THE AMERICAN CHEMICAL SOCIETY, vol. 61, no. 7, 6th July 1939, pages 1795-1796; J.H. SIMONS et al.: "Hydrogen fluoride as a condensing agent. VII. The acylation of aromatic compounds"**

(73) Proprietor: **CELANESE CORPORATION**
**1211 Avenue of the Americas**
**New York New York 10036 (US)**

(72) Inventor: **Davenport, Kenneth Gerald**
**3126 Crest Water**
**Corpus Christi Texas (US)**
Inventor: **Hilton, Charles Bruce**
**505 Williamson Place**
**Corpus Christi Texas (US)**
Inventor: **Fruchey, Olan Stanley**
**2310 Rain Tree Lane**
**Corpus Christi Texas (US)**

(74) Representative: **De Minvielle-Devaux, Ian Benedict Peter et al**
**CARPMAELS & RANSFORD 43, Bloomsbury Square**
**London WC1A 2RA (GB)**

(56) References cited:
**J.Sci.,Nat.Sci. 1975, 14(7), 5-8**

**J.Chem.Research (5) 1983, 18-19**

**Chemische Berichte (1925) 58m 36-51**

**Ullmann, 4. Aufl. Bd. II (1976), 76**

**EP 0 168 908 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

It is known to produce N-acyl-acyloxy aromatic amines, e.g. 4-acetoxy-acetanilide, by preparing the sodium salt of the corresponding N-acylhydroxy aromatic amine, e.g. N-acetyl-para-aminophenol (APAP), and reacting the sodium salt with the appropriate carboxylic acid anhydride, e.g. acetic anhydride. The N-acyl-hydroxy aromatic amine, e.g. APAP, used as the starting material for the foregoing reaction is in turn prepared by acylating the corresponding hydroxy aromatic amine, e.g. para-aminophenol, with an acylating agent such as an anhydride, e.g. acetic anhydride. However, the latter reaction may cause problems such as the difficulty of mono-acylating the hydroxy aromatic amine, oligomerization of the hydroxy aromatic amine, and color body formation.

Furthermore, when APAP is produced from para-aminophenol, nitro-benzene typically is catalytically hydrogenated and concomitantly rearranged in the presence of a platinum catalyst to produce the para-aminophenol, presenting the problem of recovering the dissolved platinum catalyst.

It is also known to prepare APAP by hydrogenating 4-nitro-chlorobenzene to a 4-chloroaniline which is then reacted with aqueous KOH to form para-aminophenol. This is then acetylated as described previously to form the N-acetyl-para-aminophenol. This process is relatively complex requiring a fair number of reaction and purification steps. Moreover, the acetylation step in this process is believed to give rise to the same problems as occurs in the acetylation step of the nitrobenzene process described previously.

The preparation of hydroxy aromatic ketones by the Fries rearrangement of aromatic esters is well-known in the art. Thus, Lewis, U.S. Patent No. 2,833,825 shows the rearrangement of phenyl or other aromatic esters to acylphenols or other hydroxy aromatic ketones using anhydrous hydrogen fluoride as catalyst. The examples of this patent are limited to the rearrangement of esters of higher fatty acids with the yields ranging from 55 to 95%.

Simons et al, Journal of the American Chemical Society, *62,* 485 and 486 (1940) show the use of hydrogen fluoride as a condensing agent for various rearrangements and at page 486 show the Fries rearrangement of phenyl acetate to obtain p-hydroxyacetophenone.

Dann and Mylius in a dissertation included as part of a series of Reports from the Institute for Applied Chemistry of the University of Erlangen, received for publication on January 7, 1954 and published in Annalen der Chemie 587 Band, pages 1 to 15 (1954), show the rearrangement of phenyl acetate in hydrogen fluoride to 4-hydroxyacetophenone, with a maximum yield of 81% after 24 hours of reaction time, and report a yield of 92% stated to be obtained by K. Weichert as reported in Angewandte Chemie 56, 338 (1943). However, Dann and Mylius suggest that the difference in yields may be at least partly due to the previous ignoring by Weichert of the accompanying 2-hydroxyacetophenone.

Dann and Mylius also disclose the reaction of phenol and glacial acetic acid in the presence of hydrogen fluoride to produce 4-hydroxyacetophenone at a yield of 61.6%. This reaction may be conventionally characterized as a Freidel-Crafts acetylation of phenol with acetic acid as the acetylating agent.

Simon et al, Journal of the American Chemical Society, *61,* 1795 and 1796 (1939) teach the acylation of aromatic compounds using hydrogen fluoride as a condensing agent and in Table 1 on page 1796 show the acetylation of phenol with acetic acid to produce p-hydroxyacetophenone in 40% yield.

Meussdoerffer et al, German Offenlegungsschrift 26 16 986 published October 27, 1977 and assigned to Bayer AG, disclose the acylation of phenolic compounds such as phenol itself with an acyl halide such as acetyl chloride to form hydroxy aromatic ketones.

Auwers et al, Chemische Berichte, *58,* 36—51, (1925) show the Beckmann rearrangement of a large number of oximes of aromatic ketones most of which are substituted acetophenones. However, the only attempted rearrangement of the oxime of a ring-unsubstituted hydroxy aromatic ketone was that of the oxime of o-hydroxyacetophenone, but no amine was formed, i.e. the attempted rearrangement was unsuccessful; see page 41.

Ganboa et al, Synthetic Communications *13*(11), 941—944 (1983) show the production of acetanilide from acetophenone by refluxing in a solution of hydroxylamine hydrochloride. There is however no suggestion of the synthesis of N-acylacyloxy aromatic amines such as 4-acetoxyacetanilide (AAA) or of the synthesis of N-acylhydroxy aromatic amines such as N-acetyl-para-aminophenol (APAP).

Pearson et al, Journal of the American Chemical Society *75* 5905—5908 (Dec. 5, 1953) disclose the formation of hydrazones from ketones by reaction with hydrazine hydrate and the rearrangement of the hydrazone to the amide by reaction with sodium nitrite and concentrated sulfuric acid. Specifically, on page 5907 Pearson et al show the rearrangement of p-hydroxyacetophenone hydrazone to p-hydroxyacetanilide, i.e. APAP.

The present invention provides a process for preparing an N-acyl-p-aminophenol or a 4-acyloxy-N-acrylanilide comprising contacting a 4-acylphenol, with hydroxylamine, or a hydroxylamine salt, and a base to form the ketoxime of said ketone, and (i) contacting said ketoxime with a Beckmann rearrangement catalyst to form an N-acyl-para-aminophenol, or (ii) contacting said ketoxime with a carboxylic acid anhydride and a Beckmann rearrangement catalyst to form a 4-acyloxy-N-acylanilide.

In one specific embodiment, N-acetyl-para-aminophenol (APAP), also known as acetaminophen, is produced from phenyl acetate, or phenol and an acetylating agent such as acetic acid, by means of an integrated process including the steps of converting the phenyl acetate, or phenol and an acetylating agent,

to 4-hydroxyacetophenone (4-HAP) by a Fries rearrangement of Friedel-Crafts acetylation respectively, and converting the 4-hydroxyacetophenone to the corresponding ketoxime with hydroxylamine or a hydroxylamine salt. The ketoxime is then subjected to a Beckmann rearrangement in the presence of a catalyst to form the N-acetyl-para-aminophenol.

In another specific embodiment, 4-acetoxyacetanilide (AAA) is produced from phenyl acetate, or phenol and an acetylating agent such as acetic acid, by means of an integrated process including the steps of converting the phenyl acetate, or phenol and an acetylating agent, to 4-hydroxyacetophenone (4-HAP) by a Fries rearrangement of Friedel-Crafts acetylation respectively, and converting the 4-hydroxyacetophenone to the corresponding ketoxime with hydroxylamine or a hydroxylamine salt. The ketoxime is then subjected to a Beckmann rearrangement and accompanying acetylation by contacting the ketoxime with acetic anhydride and a Beckmann rearrangement catalyst to form the 4-acetoxyacetanilide.

When carrying out the process of this invention using phenyl acetate as the starting material, the initial Fries rearrangement to produce 4-hydroxyacetophenone (4-HAP) from phenyl acetate is defined by equation (I):

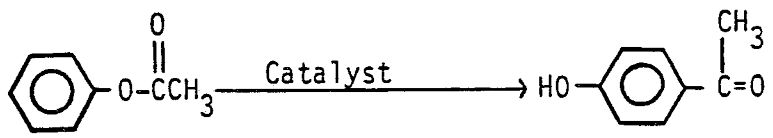


(I)

If phenol and an acetylating agent are used as the starting material, the resulting acetylation reaction to form 4-HAP is indicated by equation (II):

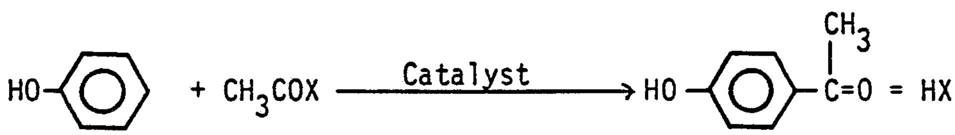


(II)

where X is the residue minus an acetyl group of compounds which are known acetylating agents. X may be, for example, hydroxy, acetoxy, or halide including fluoride, chloride, bromide, or iodine. Acetylating agents which may be used are for example, acetic acid, acetic anhydride, acetyl fluoride, acetyl chloride and acetyl bromide.

The ketoxime formation of this invention proceeds as indicated in equation (III):

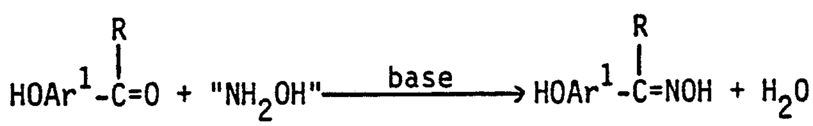


(III)

The formation of the ketone of 4-HAP, i.e. 4-HAP oxime, proceeds as in equation (IV):

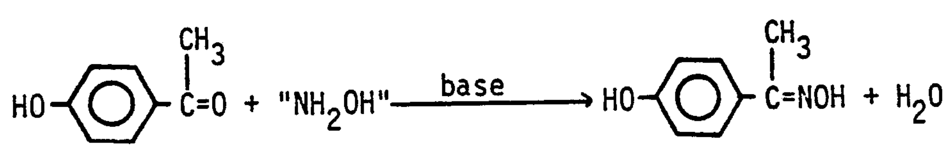


(IV)

When N-acyl-hydroxy aromatic amines are the desired product, the Beckmann rearrangement of this invention proceeds as in equation (V):

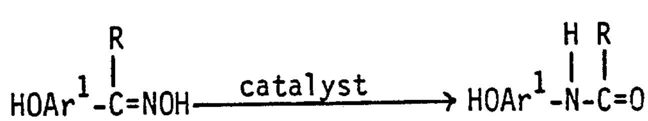


(V)

while the Beckmann rearrangement when APAP is the desired product proceeds as in equation (VI):

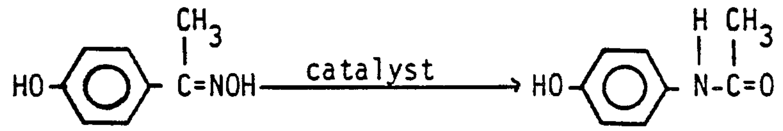

(VI)

When N-acyl-acyloxy aromatic amines are the desired product, the Beckmann rearrangement and accompanying acylation of this invention proceeds as in equation (VII):

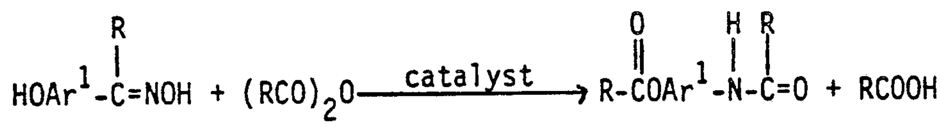

(VII)

while the Beckmann rearrangement and accompanying acylation when AAA is the desired product proceeds as in equation (VIII):

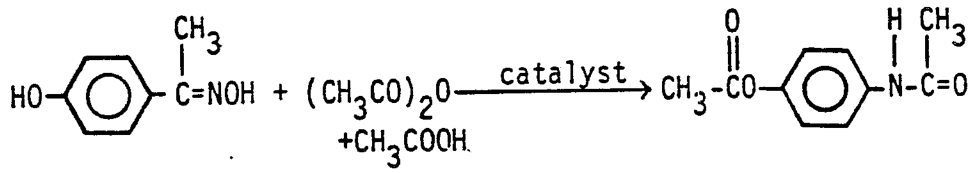

(VIII)

In equations (III), (V) and (VII), $Ar^1$ is 1,4-phenylene.

The R groups in the foregoing equations may be the same or different and are each are a monovalent organic radical containing, for example, 1 to 18 carbon atoms, preferably 1 to 4 carbon atoms. R may be, for example, alkyl, alkenyl, alkynyl, alkoxy, acyl or acyloxy containing 1 to 18 carbon atoms, either unsubstituted or substituted with radicals such as halogen, e.g. chlorine, bromine, or iodine; hydroxy; amino; sulfhydryl; or any ary radical, Ar, which may be a monovalent radical resulting from the removal of one ring hydrogen atom from benzene, naphthalene, or biphenyl, either unsubstituted or with ring hydrogens substituted with radicals such as alkyl, alkenyl, alkynyl, alkoxy or acyloxy containing 1 to 18 carbon atoms, aralkyl containing 7 to 18 carbon atoms; halogen, e.g. chlorine, bromine, or iodine; hydroxy; amino; or sulfhydryl. Ar is preferably phenyl, 2 naphthyl, 3-phenyl-phenyl or 3 methyl-phenyl. Preferably, R is the same in all occurrences in equations (III), (V), and (VII) and is methyl, ethyl, propyl, or n-butyl and most preferably methyl corresponding to the use of acetate esters and methyl ketones in the latter equations. The preferred specific hydroxy aromatic ketone used to form the oxime is 4-hydroxyacetophenone (4-HAP) and the preferred products are 4-acetoxyacetanilide (AAA) and N-acetyl-para-aminophenol (APAP).

The hydroxy aromatic ketone used to form the oxime may be prepared by any method known in the art. For example, it may be prepared by the Fries rearrangement of the corresponding aromatic ester as indicated by the following equation, which is a generalized form of equation (I), where $Ar^1$ and R have the definitions given above:

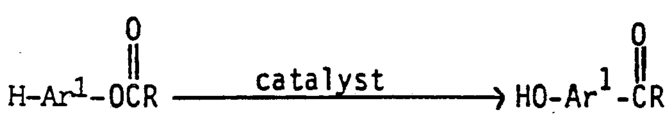

(IX)

Alternatively, a phenolic compound and an acylating agent may be reacted in a Friedel-Crafts acylation to form the hydroxy aromatic ketone, in accordance with the following equation, which is a generalized form of equation (II):

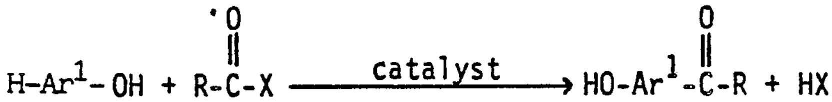

(X)

where $Ar^1$ and R have the meanings given previously and X is the residue minus the acyl group, R—CO—, of the compounds which are known acylating agents, such as hydroxy, acyloxy, e.g. acetoxy, and halide, e.g. flouride, chloride, bromide, and iodide. The phenolic compound which may be employed is phenol.

Acylating agents which may be used are for example alkanoic acids, e.g. acetic and propionic acids, alkanoic acid anhydrides, e.g. acetic and propionic anhydrides, and acyl halides, e.g. acetyl and propionyl fluorides, chlorides, and bromides. Note that although the reaction of a phenolic compound and an acylating agent is characterized herein as a "Friedel-Crafts acylation, "no opinion as to the mechanism of

reaction should be implied by this characterization.

The catalyst for both of the foregoing reactions is preferably hydrogen fluoride but any other catalyst known in the art to be effective for the Fries and Friedel-Crafts reactions may be used, e.g. aluminum chloride, zinc chloride, or boron trifluoride.

In carrying out the reaction, the aromatic ester or phenolic compound and acylating agent, catalyst and if desired when an aromatic ester is the starting material, an additive for the reaction such as acetic anhydride or acetic acid, may be charged to a corrosion-resistant reactor and the mixture maintained at a temperature, for example, of about 20 to about 100°C for a period, for example, of about ½ to about 4 hours, at a pressure, for example, of about 50 to about 500 psi (3.4 to 34 bar). If HF is used as the catalyst it may be charged as a liquid or a gas using technologies of handling well-known to those skilled in the art. In carrying out the reaction, an inert gas such as nitrogen may be used to keep the reaction space under the desired pressure and sufficient HF in contact with the reacting liquid. An excess of HF is generally used, for example, about 7 to about 75 moles per mole of aromatic ester or phenolic compound initially present in the reaction zone. If AAA or APAP is the desired product of the reaction, the starting material if a Fries rearrangement is employed will be phenyl acetate while phenol and an acetylating agent such as acetic acid is the starting material if a Friedel-Crafts acylation is utilized. In both cases, the starting material is converted to 4-HAP which is in turn converted by the process of this invention to AAA or APAP.

The conversion of hydroxy aromatic ketones, e.g. 4-HAP, into N-acylacyloxy aromatic amines, e.g. AAA, or into N-acyl-hydroxy aromatic amines, e.g., APAP, is accomplished by first forming the ketoxime from the hydroxy aromatic ketone as indicated by equations (III) and (IV), by contacting the ketone with hydroxylamine or a hydroxylamine salt, e.g. hydroxylamine hydrochloride, hydroxylamine sulfate, hydroxylamine bisulfate, or hydroxylamine phosphate, and a base, e.g. ammonium hydroxylide, potassium hydroxide, sodium hydroxide, or lithium hydroxide in an amount, for example, of 1 to 3 moles per mole of hydroxylamine, at a temperature, for example of 0 to 60°C for a period, for example, of 1 to 4 hours. Any pressure may be used, e.g. 80 mm of mercury to 10 atmospheres absolute (0.1 bar to 10.1 bar). The reaction is preferably carried out in an aqueous or alcoholic medium, i.e. in the presence of water and/or an alcohol such as methanol, ethanol, or isopropanol.

As discussed above, in accordance with one embodiment of the invention, the ketoxime may be converted into the corresponding N-acyl-hydroxy aromatic amine by a Beckmann rearrangement as shown in equations (V) and (VI), by contacting the ketoxime with a catalyst for the reaction at a temperature, for example of −70°C to 118°C for a period for example of ten minutes to 4 hours. The pressure is not critical and may be, for example, in the range of 80 mm of mercury to 10 atmospheres absolute (0.1 bar to 10.1 bar). Preferably, the rearrangement is conducted at a temperature of from about −70°C to about 40°C and at a molar ratio of ketoxime to catalyst from about 1:0.001 to about 1:0.1, for a reaction time of about ten minutes to about two hours. Any Beckmann rearrangement catalyst may be used, as for example, an acid, e.g. mineral acid such as sulfuric or hydrochloric acid, an organic acid such as trifluoroacetic acid, paratoluenesulfonic acid, benzenesulfonic acid or methanesulfonic acid, an acidic ion-exchange resin such as Amberlyst (trade mark) 15 or Nafion (trade mark) 501 which are sulfonic acid ion-exchange resins, or thionyl chloride in liquid sulfur dioxide, diethyl ether, ethylacetate, acetone, tetrahydrofuran, or methylene chloride. Preferably the Beckmann rearrangement is conducted with thionyl chloride in liquid sulfur dioxide. The reaction may be advantageously carried out in the presence of the glacial carboxylic acid corresponding to the N-acyl group of the desired product which will ordinarily yield the hydroxy derivative. The total amount of glacial carboxylic acid is not critical but is usally present such that the ketoxime concentration is in the range of 2 to 50% by weight at the start of the reaction.

In accordance with another embodiment of the invention, the ketoxime may be converted into the corresponding N-acyl-acyloxy aromatic amine by a Beckmann rearrangement and accompanying acylation as shown in equations (VII) and (VIII), by contacting the ketoxime with the appropriate carboxylic acid anhydride and a Beckmann rearrangement catalyst at a temperature, for example of 0 to 118°C for a period for example of 1 to 4 hours. As defined in the foregoing equations, any of a broad class of anhydrides may be used but the anhydride is preferably that of an alkanoic acid containing 2 to 4 carbon atoms, e.g. acetic anhydride, propionic anhydride, or n-butyric anhydride. The pressure is not critical and may be, for example, in the range of 80 mm of mercury to 10 atmospheres absolute (0.1 to 10.1 bar). Again, any Beckmann rearrangement catalyst may be used, as discussed above. The reaction may be advantageously carried out in the presence of the glacial carboxylic acid corresponding to the anhydride employed in the reaction in an amount, for example up to 50% by weight of the anhydride. The total amount of glacial carboxylic acid is not critical but the total amount of anhydride or anhydride/acid mixture is such that the ketoxime concentration is in most cases in the range of about 2 to 50% by weight at the start of the reaction.

The following examples further illustrate the invention.

## Example 1

This example illustrates the preparation of 4-hydroxyacetophenone by the Fries rearrangement of phenyl acetate using hydrogen fluoride as catalyst.

To a 300 cc Hastelloy C autoclave was charged 40.8 g (0.3 mol) of phenyl acetate. The autoclave was sealed, immersed in a Dry Ice/isopropanol bath and cooled internally to −45°C, and evacuated to ca. 100 Torr (0.13 bar). Addition of 120 g (6.0 mol) of anhydrous hydrogen fluoride was performed in a manner

such as that the internal temperature of the autoclave did not exceed 0°C. The internal pressure of the reactor was then adjusted to 0 psig (1.1 bar) with nitrogen. The contents of the autoclave were stirred and heated to 75°C for 1 h. The hydrogen flouride was vented over a 45 min period at ca. 45°C. The mixture was poured onto 25 g of ice and neutralized with 45% potassium hydroxide solution. The aqueous mixture was extracted with ethyl acetate. The organic fraction was then dried over anhydrous magnesium sulfate, filtered, and the solvent was removed on a rotary evaporator to yield 44.0 g of a dark green solid corresponding to 99.9% conversion of phenyl acetate and 94.3% selectivity of 4-hydroxyacetophenone.

Example 2

This example illustrates the preparation of 4-hydroxyacetophenone by the Fries rearrangement of phenyl acetate using hydrogen fluoride as catalyst with acetic anhydride as additive.

To a 300 cc Hastelloy C autoclave were added 30.6 grams (0.3 mole) of acetic anhydride. The autoclave was cooled to −50°C and evacuated to 5 Torr (0.007 bar) whereupon 120 g (6.0 mole) of anhydrous hydrogen fluoride was transferred from a cylinder to the autoclave. After the transfer of hydrogen fluoride was completed, the internal temperature and the internal pressure of the autoclave was adjusted to −50°C and 1.1 bar using nitrogen, respectively. To the stirred autoclave was added 81.6 g (0.6 mol) of phenyl acetate at such a rate that the temperature of the mixture did not exceed −23°C. Upon completion of phenyl acetate addition, the contents were warmed to 50°C and stirred for 3 h during which time a pressure of ca. 40 psig (3.9 bar) was generated. At the end of the run, the hydrogen fluoride was vented through a caustic scrubber and the contents of the autoclave were poured onto ca. 30 g of ice. The pH of the mixture was adjusted to 6.5 using 45% potassium hydroxide and the mixture was then extracted with 75 ml of ethyl acetate (3×). The organic solution was dried over anhydrous magnesium sulfate, filtered, and the solvent was removed using a rotary evaporator.

The reaction proceeded with 98.1% conversion of phenyl acetate and with the following selectivities: phenol 1%, 4-hydroxyacetophenone (4-HAP) 82.3%; 2-hydroxyacetophenone (2-HAP) 4.3%; 3-hydroxyacetophenone (3-HAP) 0.1%; 4-acetoxyacetophenone (4-AAP) 3.8%; and 4-(4'-hydroxyphenyl)acetophenone (HPAP) 0.4%.

Example 3

This example describes the formation of 4-hydroxyacetophenone by the Fries rearrangement of phenyl acetate using hydrogen fluoride as catalyst and acetic acid as additive.

The procedure for Example 2 was repeated except that 18 grams (0.3 mole) of acetic acid rather than acetic anhydride were charged to the reactor before it was cooled and charged with the hydrogen fluoride. A conversion of 99.0% of phenyl acetate was obtained with the following selectivities: phenol 3.3%; acetic acid 0.8%; 4-HAP 80.8%; 3-HAP 0; 2-HAP 5.8%; 4-AAP 0.3%; and HPAP 0.3%.

Example 4

This example illustrates the preparation of 4-hydroxyacetophenone (4HAP) by the Friedel-Crafts acetylation of phenol with acetic acid as the acetylating agent.

Phenol (9.4 g, 0.1 moles) and acetic acid (12.0 g, 0.2 moles) were charged to a 300 ml Hastelloy C autoclave at room temperature. The reactor was evacuated and cooled to −20°C. HF (100 g, 5 moles) was then transferred into the reactor. The reactor was heated to 80°C and maintained for 1 hour at reaction temperature. At the end of the reaction the reactor was cooled to 20°C and the excess HF was vented to a KOH scrubber. Ethyl acetate was added to the contents of the reactor. The mixture was then neutralized with 45% aqueous KOH. The resulting organic phase was separated, dried over $MgSO_4$ and evaporated to afford a yellow solid which contained 13.1 g (0.096 moles) of 4-HAP.

Example 5

This example illustrates the formation of 4-hydroxyacetophenone oxime from 4-hydroxyacetophenone and hydroxylamine hydrochloride.

A solution was prepared by adding 13.6 g (0.1 mol) of 4-hydroxyacetophenone 7.6 g (0.11 mol) of hydroxylamine hydrochloride, and 10 g of water to 40 mL of ethanol. To the solution was added 5.0 g of 30% ammonium hydroxide which was then heated at reflux for 2 h. The ethanol was removed on a rotary evaporator to yield a yellow oil. An extractive work-up afforded 15.1 g (99%) of 4-hydroxyacetophenone oxime.

Example 6

This example illustrates the formation of 4-hydroxyacetophenone oxime from 4-hydroxyacetophenone and hydroxylamine sulfate.

A solution was prepared by adding 20.4 g (0.15 mol) of 4-hydroxyacetophenone and 13.0 g (0.08 mol) of hydroxylamine sulfate to 100 mL of water at 70°C. To the solution was aded 16.3 mL of 30% ammonium hydroxide which was then heated at reflux for 0.5 h. White crystals formed upon cooling yielding 21.0 g (92.6%) of 4-hydroxyacetophenone oxime.

### Example 7

This example illustrates the formation of 4-hydroxyacetophenone oxime from 4-hydroxyacetophenone and hydroxylamine phosphate.

A solution was prepared by adding 20.4 g (0.15 mol) of 4-hydroxyacetophenone and 12.9 g (65.6 mmol) of hydroxylamine phosphate to 100 mL of water at 70°C. To the solution was added 16.3 mL of 30% ammonium hydroxide which was then heated at reflux for 0.5 h. White crystals formed upon cooling yielding 21.0 g (92.6%) of 4-hydroxyacetophenone oxime.

### Example 8

This example illustrates the formation of 4-acetoxyacetanilide (AAA) by the Beckmann rearrangement and accompanying acetylation of 4-hydroxyacetophenone oxime using an acidic ion-exchange resin as catalyst.

A mixture of 3.0 g (22.0 mmol) of 4-hydroxyacetophenone oxime, 3.0 of Amberlyst (trade mark) 15 (a sulfonic acid ion-exchange resin made by Rohm & Haas), and 75 mL of a mixture of glacial acid and acetic anhydride (1:1) was heated at reflux under nitrogen for 4 h. The ion-exchange resin was then removed and the acetic acid/acetic anhydride was distilled in vacuo to yield yellow-white crystals. The crystals were dissolved in ethyl acetate and treated with activated carbon and anhydrous magnesium sulfate. The mixture was filtered and the solvent was removed on a rotary evaporator to yield 3.4 g (80.4%) of yellow crystals of 4-acetoxyacetanilide (AAA).

### Example 9

This example illustrates the formation of 4-acetoxyacetanilide (AAA) by the Beckmann rearrangement and accompanying acetylation of 4-hydroxyacetophenone oxime using methanesulfonic acid as catalyst.

A solution of 10 g (66.2 mmol) of 4-hydroxyacetophenone oxime, 1.6 of 70% methanesulfonic acid, 50 g of acetic anhydride and 100 g of glacial acetic acid was heated at reflux under nitrogen for 2 h. Rotary evaporation of the solution yielded 17.0 g of light brown crystals. Recrystallization from water yielded 6.7 g (52.4%) of 4-acetoxyacetanilide (AAA). The mother liquor contained 32.0% of AAA for a total yield of 84.4%.

### Example 10

This example illustrates the formation of 4-acetoxyacetanilide (AAA) by the Beckmann rearrangement and accompanying acetylation of 4-hydroxyacetophenone oxime using phosphoric acid ($H_3PO_4$) as catalyst.

To a mixture of 100 g of glacial acetic acid, 50 g of acetic anhydride, and 3.6 g of 85% $H_3PO_4$, sparged with nitrogen for 30 minutes, was added 10 g of 4-hydroxyacetophenone oxime. The mixture was heated at reflux for 1 hour under a nitrogen atmosphere, then cooled to room temperature and neutralized with 13% $Na_2CO_3$. The mixture was evaporated to dryness using a rotary evaporator and the solid was dissolved in 200 g of boiling water. After hot filtration, the solution was allowed to cool and stand overnight. The ensuing white crystals were collected, washed with 20 mL of water, and dried in a vacuum oven (60°C/100 mm Hg (0.13 bar)) for 2 hours. Upon drying, 9.4 g (73.9%) of white crystalline plates of 4-acetoxy-acetanilide having a melting point of 148—150°C was obtained. An additional 0.8 g of AAA and 1.5 of N-acetyl-paraaminophenol (APAP) were reclaimed from the mother liquor.

### Example 10A

The procedures of Examples 8 to 10 may also be used to prepare N-propionyl-(4-propionoxyphenyl) amine from phenyl propionate or phenol and propionic acid, and propionic anhydride; and N-n-butyryl-(4-n-butyroxyphenyl) amine from phenyl n-butyrate or phenol and n-butyric acid, and n-butyric anhydride, in the first and second reactions respectively.

The N-acyl-acyloxy aromatic amines, e.g. AAA, of this invention may be utilized as monomers in the preparation of poly(ester-amide)s capable of forming an anisotropic melt phase and suitable for being formed into shaped articles such as molded articles, fibers and films, as shown, for example in U.S. Patent Nos. 4,330,457; 4,339,375; 4,341,688; 4,351,918; and 4,355,132.

The N-acyl-acyloxy aromatic amines of this invention, e.g. AAA, may also be hydrolyzed to form the corresponding N-acyl-hydroxy aromatic amine, e.g. N-acetyl-para-aminophenyl (APAP) which is one of the most widely used over-the-counter analgesics. The following example illustrates this process:

### Example 11

A mixture of 5 g (25.9 mmol) of 4-acetoxyacetanilide (AAA), 1.4 g of 70% methanesulfonic acid, and 50 g of water was heated at reflux for 1 h. Upon cooling, white crystals formed. Analysis (GLC) of the crystals as well as the aqueous solution indicated 90% conversion of the AAA to N-acetyl-para-aminophenol (APAP).

### Example 12

This example illustrates the formation of N-acetyl-para-aminophenol by the Beckmann rearrangement of 4-hydroxyacetophenone oxime using an acidic ion-exchange resin as catalyst.

A mixture of 3.0 g of Amberlyst (trade mark) 15 (a sulfonic acid ion-exchange resin made by Rohm & Haas), 3.0 g (22.0 mmol) of 4-hydroxyacetophenone oxime, and 50 mL of acetic acid was heated at reflux under nitrogen for 2 h. The ion exchange resin was then removed and the acetic acid was distilled in vacuo to afford an orange residue. The residue was dissolved in ethanol and treated with activated carbon and anhydrous magnesium sulfate. Removal of the ethanol using a rotary evaporator produced 2.9 g of a yellow oil, which upon drying afforded 2.0 g (66.7%) of N-acetyl-para-aminophenol.

Example 13

This example illustrates the formation of N-acetyl-para-aminophenol by the Beckman rearrangement of 4-hydroxyacetophenone oxime using triflouroacetic acid as catalyst.

A solution of 10 g (66.2 mmol) of 4-hydroxyacetophenone oxime and 75 g of trifluoroacetic acid was heated at reflux under a nitrogen atmosphere. The trifluoroacetic acid was then removed in a rotary evaporator to afford 14.7 g of oil which was dissolved in 100 mL of water. Upon cooling to 0°C for 0.5 h, crystallization occurred. Filtration and drying of the crystals yielded 7.1 g (71%) of N-acetyl-para-aminophenol.

Example 14

This example illustrates the formation of N-acetyl-para-aminophenol by the Beckmann rearrangement of 4-hydroxyacetophenone oxime using thionyl chloride in liquid sulfur dioxide as catalyst.

A pressure bottle (cooled in a $Co_2$/acetone bath) was charged with 50 mL of $SO_2$, 0.05 mL of $SOCl_2$, and 15 g of 4-hydroxyacetophenone oxime. The $CO_2$/acetone bath was removed and the contents of the pressure bottle stirred for 1.5 h at room temperature. The $SO_2$ was then vented and the crystals washed from the pressure bottle with 50 mL of warm water. The pH of the aqueous slurry was adjusted to 6.5 by dropwise addition of 30% $NH_4OH$. The slurry was cooled in an ice bath and then filtered. The filtered crystals were washed with 10 mL of ice water and dried overnight in a vacuum oven (60°C/100 mm Hg (0.13 bar)) yielding 13.3 g (88.7%) of white crystals of N-acetyl-para-aminophenol having a melting point of 166.5—170°C.

Example 15

The procedures of Examples 12 through 14 may also be used to prepare N-propionyl-para-aminophenol from phenyl propionate or phenol and propionic acid; and N-n-butyryl-para-aminophenol from phenyl n-butyrate or phenol and n-butyric acid.

Example 16

A 250 ml pressure bottle was first cooled in a Dry Ice/acetone bath and was then charged with 50 ml of $SO_2$ (via vacuum transfer), 0.05 mL of $SOCl_2$, and 15 g of 4-hydroxyacetophenone oxime. The Dry Ice/acetone bath was removed and the contents of the pressure bottle stirred for 1.5 h at room temperature. The $SO_2$ was then vented and the crystals washed from the pressure bottle with 50 mL of warm water. The pH of the aqueous slurry was adjusted to 6.5 by the dropwise addition of concentrated ammonium hydroxide. The slurry was cooled in an ice bath and then filtered. The filtered crystals were washed with 10 mL of ice water and dried overnight in a vacuum oven at 60°C, yielding 13.3 g of white N-acetyle para-aminophenol crystals with a melting point of 166.5—170°C.

Example 17

The same general procedure as Example 16 was employed except that tap water (24°C) was used to wash the crystals from the pressure bottle. Also, the amount of $SOCl_2$ was increased to 0.1 ml and the reaction time was decreased to 25 minutes. Off-white crystals of N-acetyl para-aminophenol (13.7 g) were recovered with a melting point of 165—169°C.

Example 18

This example illustrates the preparation of N-acetyl para-aminophenol by the Beckmann rearrangement of 4-hydroxyacetophenone using thionyl chloride as catalyst in diethyl ether.

A 250 ml round-bottom flask equipped with a reflux condenser and addition funnel was charged with 5 g of 4-hydroxyacetophenone oxime dissolved in 50 mL of anhydrous diethyl ether. A solution of 0.5 mL of thionyl chloride in 15 mL of ether was added dropwise from the addition funnel. The contents of the flask were stirred during the addition and for an additional 30 minutes after completion of the addition. The ether was then removed on a rotovap. The solid residue was then dissolved in 25 mL of hot water. The pH of the solution was adjusted to about 6.5 with ammonium hydroxide and subsequently the solution was cooled in an ice bath. The crystals which were formed were filtered and washed with approximately 10 mL of ice water and then dried in a vacuum oven at 65°C overnight. Brown crystals of N-acetyl para-aminophenol (1.1 g) were obtained with a melting point of 161—2°C.

Example 19

This example illustrates the preparation of N-acetyl para-aminophenol from 4-hydroxyacetophenone, using thionyl chloride as the catalyst in ethyl acetate.

The same procedure as Example 18 was employed except that ethyl acetate was used as the solvent instead of diethyl ether. Light brown crystals of N-acetyl para-aminophenol (1.9 g) with a melting point of 158—161°C were recovered.

Example 20

This example illustrates the preparation of N-acetylated para-aminophenol from 4-hydroxyacetophenone using thionyl chloride as catalyst in acetone.

The same procedure as Example 18 was employed except that acetone was used as the solvent instead of diethyl ether. Brown crystals of N-acetyl para-aminophenol (3.7 g) with a melting point of 159—161°C were recovered.

Example 21

This example illustrates the preparation of N-acetyl para-aminophenol from 4-hydroxyacetophenone oxime using thionyl chloride as catalyst in tetrahydrofuran.

The same procedure as Example 18 was employed except that tetrahydrofuran was used as the solvent instead of diethyl ether. Tan crystals of N-acetyl para-aminophenol (2.5 g) with a melting point of 156—8°C were recovered.

Example 22

This example illustrates the preparation of N-acetyl para-aminophenol from 4-hydroxyacetophenone oxime using thionyl chloride as the catalyst in methylene chloride.

The same procedure as Example 18 was employed but methylene chloride was used as the solvent instead of diethyl ether. Dark brown crystals of N-acetyl para-aminophenyl (2.7 g) with a melting point of 152—156°C were obtained.

Example 23

This example illustrates the preparation of N-acetyl para-aminophenol from 4-hydroxyacetophenone oxime using thionyl chloride as catalyst in acetone, under vacuum conditions.

The same procedure as Example 18 was employed except that acetone was used as the solvent instead of diethyl ether and the system was run under vacuum (360 mm Hg (0.48 bar)). Tan crystals of N-acetyl para-aminophenol (3.6 g) with a melting point of 162—164°C were obtained.

Example 24

This example illustrates the fact that the process of the present invention is capable of producing nearly quantitative yields of the desired N-acyl-hydroxy aromatic amine.

The same general procedure as Example 17 was employed except that the filtrate was also analysed for N-acetyl para-aminophenol to determine the actual product yield. The recovered solid weight 13.7 g and the filtrate contained an additional 0.7 g of N-acetyl para-aminophenol. Therefore, a yield of 97 percent was realized.

**Claims**

1. A process for preparing an N-acyl-p-aminophenol or a 4-acyloxy-N-acryl-anilide comprising contacting 4-acylphenol with hydroxylamine, or a hydroxylamine salt, and a base to form the ketoxime of said 4-acylphenol, and (i) contacting said ketoxime with a Beckmann rearrangement catalyst to form N-acyl-para-aminophenol, or (ii) contacting said ketoxime with a carboxylic acid anhydride and a Beckmann rearrangement catalyst to form 4-acyloxy-N-acyl-anilide.

2. The process of claim 1 wherein said 4-acylphenol is 4-hydroxyacetophenone, said ketoxime is 4-hydroxyacetophenone oxime, and said N-acyl-para-aminophenol is N-acetyl-para-aminophenol.

3. The process of claim 2 wherein the ketoxime is contacted with a catalytic amount of thionyl chloride in a solvent selected from liquid sulfur dioxide, diethyl ether, ethylacetate, acetone, tetrahydrofuran, and methylene chloride.

4. The process of claim 3 wherein the solvent is sulfur dioxide and the rearrangement is conducted at a temperature of from −70°C to 40°C and at a pressure of from 0.1 bar to 10 bar.

5. The process of claim 1 wherein said 4-acylphenol is 4-hydroxyacetophenone, said ketoxime is 4-hydroxyacetophenone oxime, said anhydride is acetic anhydride, and said 4-acyloxy-N-acyl-anilide is 4-acetoxyacetanilide.

6. The process of claim 5 wherein said 4-acetoxyacetanilide is hydrolyzed to form N-acetyl-para-aminophenol.

7. The process of any of claims 1 to 6 also comprising contacting an ester of phenols and a carboxylic acid with a Fries arrangment catalyst to form said 4-acylphenol.

8. The process of claim 7 wherein said Fries rearrangement catalyst is hydrogen fluoride.

9. The process of claim 7 or 8 wherein the ester is phenylacetate.

10. The process of any of claims 1 to 6 also comprising contacting phenol and an acylating agent with a Friedel-Crafts catalyst to form said 4-acylphenol.

11. The process of claim 10 wherein said Friedel-Crafts catalyst is hydrogen fluoride.
12. The process of claim 10 or 11 wherein the acylating agent is acetic acid.

**Patentansprüche**

1. Verfahren zur Herstellung eines N-Acyl-p-aminophenols oder eines 4-Acyloxy-N-acylanilids, dadurch gekennzeichnet, daß man ein 4-Acylphenol mit Hydroxylamin oder einem Hydroxylaminsalz und einer Base in Berührung bringt, um das Ketoxim des 4-Acylphenols zu bilden, und (i) das Ketoxim mit einem Beckmann-Umlagerungskatalysator in Berührung bringt, um das N-Acyl-p-aminophenol zu bilden, oder (ii) das Ketoxim mit einem Carbonsäureanhydrid une einem Beckmann-Umlagerungskatalysator in Berührung bringt, um das 4-Acyloxy-N-acylanilid zu bilden.
2. Verfahren nach Anspruch 1, dadurch gekennzeichnet, daß das 4-Acylphenol 4-Hydroxyacetophenon, das Ketoxim 4-Hydroxyacetophenonoxim und das N-Acyl-p-aminophenol N-Acetyl-p-aminophenol ist.
3. Verfahren nach Anspruch 2, dadurch gekennzeichnet, daß das Ketoxim mit einer katalytischen Menge an Thionylchlorid in einem Lösungsmittel, ausgewählt aus flüssigem Schwefeldioxid, Diethylether, Ethylacetat, Aceton, Tetrahydrofuran und Methylenchlorid, in Berührung gebracht wird.
4. Verfahren nacn Anspruch 3, dadurch gekennzeichnet, daß das Lösungsmittel Schwefeldioxid ist und die Umlagerung bei einer Temperatur von −70°C bis 40°C bei einem Druck von 0,1 bar bis 10 bar durchgeführt wird.
5. Verfahren nach Anspruch 1, dadurch gekennzeichnet, daß das 4-Acylphenol 4-Hydroxyacetophenon, das Ketoxim 4-Hydroxyacetophenoxim, das Anhydrid Essigsäureanhydrid und das 4-Acyloxy-N-acylanilid 4-Acetoxyacetanilid ist.
6. Verfarhen nach Anspruch 5, dadurch gekennzeichnet, daß das 4-Acetoxyacetanilid hydrolysiert wird, um N-Acetyl-p-aminophenol zu bilden.
7. Verfarhen nach einem der Ansprüch 1 bis 6, weiters dadurch gekennzeichnet, daß man einen Ester oder ein Phenol und eine Carbonsäure mit einem Fries-Umlagerungskatalysator umsetzt, um das 4-Acylphenol zu bilden.
8. Verfahren nach Anspurch 7, dadurch gekennzeichnet, daß der Fries-Umlagerungskatalysator Fluorwasserstoff ist.
9. Verfahren nach Anspruch 7 oder 8, dadurch gekennzeichnet, daß der Ester Phenylacetat ist.
10. Verfahren nach einem der Ansprüche 1 bis 6, weiters dadurch gekennzeichnet, daß man Phenol un ein Acylierungsmittel mit einem Friedel-Crafts-Katalysator umsetzt, um das 4-Acylphenol zu bilden.
11. Verfahren nach Anspruch 10, dadurch gekennzeichnet, daß der Friedel-Crafts-Katalysator Fluorwasserstoff ist.
12. Verfahren nach Anspruch 10 oder 11, dadurch gekennzeichnet, daß das Acylierungsmittel Essigsäure ist.

**Revendications**

1. Procédé pour préparer un N-acyl-p-aminophénol ou une 4-acyloxy-N-acyl-anilide consistant à mettre en contact du 4-acylphénol avec de l'hydroxylamine, ou un sel d'hydroxylamine, et une base pour former le cétoxime dudit 4-acylphénol, et (i) mettre en contact ladite cétoxime avec un catalyseur de réarrangement de Beckmann pour former du N-acyl-para-aminophénol, ou (ii) mettre en contact ladite cétoxime avec un acide carboxylique anhydre et un catalyseur de réarrangement de Beckmann pour former de la 4-acyloxy-N-acyl-anilide.
2. Procédé selon la revendication 1, dans lequel le 4-acylphénol est la 4-hydroxyacétophénone, ladite cétoxime est la 4-hydroxyacétophénone oxime, et ledit N-acyl-para-aminophénol est le N-acétyl-para-aminophénol.
3. Procédé selon la revendication 2, dans lequel la cétoxime est mise en contact avec une quantité catalytique de chlorure de thionyle dans un solvant choisi parmi le dioxyde de soufre liquide, le diéthyl éther, l'éthyl acétate, l'acétone, le tétrahydrofuranne et le chlorure de méthylène.
4. Procédé selon la revendication 3, dans lequel le solvant est du dioxyde sourfre et le réarrangement est conduit à une température de −70°C à 40°C et à une pression de 0,1 bar à 10 bars.
5. Procédé selon la revendication 1, dans lequel ledit 4-acylphénol est la 4-hydroxyacétophénone, ladite cétoxime est la 4-hydroxyacétophénone oxime, ledit anhydride est l'anhydride acétique et ladite 4-acyloxy-N-acyl-anilide est la 4-acétoxyacétanilide.
6. Procédé selon la revendication 5, dans lequel ladite 4-acétoxyacétanilide est hydrolysée pour former du N-acétyle-para-aminophénol.
7. Procédé selon l'une quelconque des revendications 1 à 6 consistant également à mettre en contact un ester de phénol et un acide carboxylique avec un catalyseur de réarrangement de Fries pour former ledit 4-acylphénol.
8. Procédé selon la revendication 7, dans lequel le catalyseur de réarrangement de Fries est du fluorure d'hydrogène.
9. Procédé selon la revendication 7 ou 8, dans lequel l'ester est du phényl acétate.

10. Procédé selon l'une quelconque des revendications 1 à 6 consistant également à mettre en contact du phénol et un agent d'acylation avec un catalyseur de Friedel-Crafts pour former ledit 4-acylphénol.

11. Procédé selon la revendication 10, dans lequel ledit catalyseur de Friedel-Crafts est du fluorure d'hydrogène.

12. Procédé selon la revendication 10 ou 11, dans lequel l'agent d'acylation est l'acide acétique.